# EUROPEAN PATENT APPLICATION

(11) **EP 4 148 129 A1**
(43) Date of publication of application: **15.03.2023**
(21) Application number: 21799929.1
(22) Date of filing: 30.03.2021
(51) Int. Cl.: C12N 15/10, C12N 15/63

(54) **DIRECTED EVOLUTION METHOD BASED ON PRIMARY AND SECONDARY REPLICON OF GEMINI VIRUS**

(30) Priority: 07.05.2020 CN 202010376936
(71) Applicant: Suzhou Qi Biodesign biotechnology Company Limited, Jiangsu 215127 (CN)
(72) Inventor: GAO, Caixia, Beijing 100101 (CN); ZHU, Haocheng, Beijing 100101 (CN)
(74) Representative: Nederlandsch Octrooibureau
(86) International application number: PCT/CN2021/083949
(87) International publication number: WO 2021/223545

(57) **Abstract**

The present invention belongs to the field of genetic engineering. Specifically, the present invention relates to a directed evolution method based on geminivirus. More specifically, the present invention relates to a directed evolution method for in vivo screening of a genetic element in a plant cell by using primary and secondary replicons of geminivirus.

## Description

### Technical Field

The present invention belongs to the field of genetic engineering. Specifically, the present invention relates to a directed evolution method based on geminivirus. More specifically, the present invention relates to a directed evolution method for in vivo screening of a genetic element in a plant cell by using primary and secondary replicons of geminivirus.

### Background Art

In the long history, organisms constantly produce variations, some of which are conducive to the survival of the organisms, while some of which are harmful to the survival of the organisms. Under the pressure of natural selection, the variations that are conducive to the survival of the organisms are retained and enriched, while those that are unfavorable are eliminated, this process is called evolution.

According to this principle, in the field of modern molecular biology, researchers simulate this process in the laboratory, artificially create a large number of mutations, give targeted selection pressure according to the required functions and purposes, and screen out genotypes with desired properties. This simulated evolution at the molecular level is called directed evolution.The directed evolution may modify a protein in the case of unknown structural information and mechanism of action of the target protein. Therefore, the directed evolution is an effective method to obtain new functional proteins in molecular biology research at present.

Up to now, many directed evolution systems are already reported. The main systems are a multiple automated genome engineering (MAGE) system developed by George M. Church et al. in 2009, a phase-assisted continuous evolution (ACE) system developed by David. Liu et al. in 2011, and an EvolvR system developed by John E. Dueber et al. in 2018 and the like. These systems may work efficiently, and complete the evolution of many genetic tools, such as xCas9.However, there is not a high-efficient and high-throughput directed evolution system based on a higher plant at present.

The following factors make it urgent to establish a directed evolution system working in a plant cell. First, different working temperatures and pH are required in plants. At present, most tools used in the plants come from Escherichia coli or mammals, the living temperature of Escherichia coli and mammals is generally 37°C, and the optimal working temperature of proteins coming from the two is also generally 37°C, but the optimal growth temperature of most of the plants is 20-25°C; andsimilarly, pH of an animal cell is generally 7.2-7.4, pH of Escherichia coli is 7.0-7.5, and pH of a plant cytoplasmic matrix is 5.6-5.9. As a result of the above, the proteins coming from Escherichia coli and mammals, or products obtained from the directed evolution system depending on Escherichia coli and mammals may not work as efficiently in the plants as before due to thermodynamic and chemical factors. Second, the plant cell has special anatomical structures. Compared with Escherichia coli, the plant, as a eukaryote, has a cell nucleus, an endoplasmic reticulum, and other structures in its cell; and compared with the animal cell, the plant cell has a chloroplast, a cell wall and other structures, and it is difficult for some proteins related to these structures to evolve in Escherichia coli or mammal systems. Third, the plant cell has a unique cellular regulatory network. In the long evolutionary process, a complex regulatory network is formed between various genetic elements in the cell, and there are great differences between prokaryotes and eukaryotes, and between the plant cells and animal cells. Since any elements are unlikely to be independent of this network, products obtained by the directed evolution system outside a plant cell system may not work effectively in a plant system. However, the elements in the plant cell regulatory network may not achieve the directed evolution in other biological systems. For the above several reasons, some elements that may work efficiently in the mammal or Escherichia coli system, such as eAPOBEC3A reported by J. Keith Joung et al., do not show activity in the plant cells. Therefore, a directed evolution system based on a plant cell needs to be developed in this field.

However, at present, although researchers attempt to perform the directed evolution in the plants in several jobs, these systems are difficult to be applied and popularized. The reason is that some inherent factors in the plants hinder the development of related research. Firstly, many existing directed evolution systems rely on high-frequency homologous recombination in vivo, but the efficiency of homologous recombination in the plants is less than 0.1%. Secondly, bacteria, yeast and animal cells may all prepare cell lines efficiently. However, at present, only a few plant species may prepare the cell lines, and the repeatability thereof is relatively low. Thirdly, there is a lack of a high-efficient transformation system in the plants, so that the high-throughput research in the plants needs to pay a large amount of work. These factors greatly hinder the development of the directed evolution system in the plants, and it is difficult to make a substantial change in a short time.

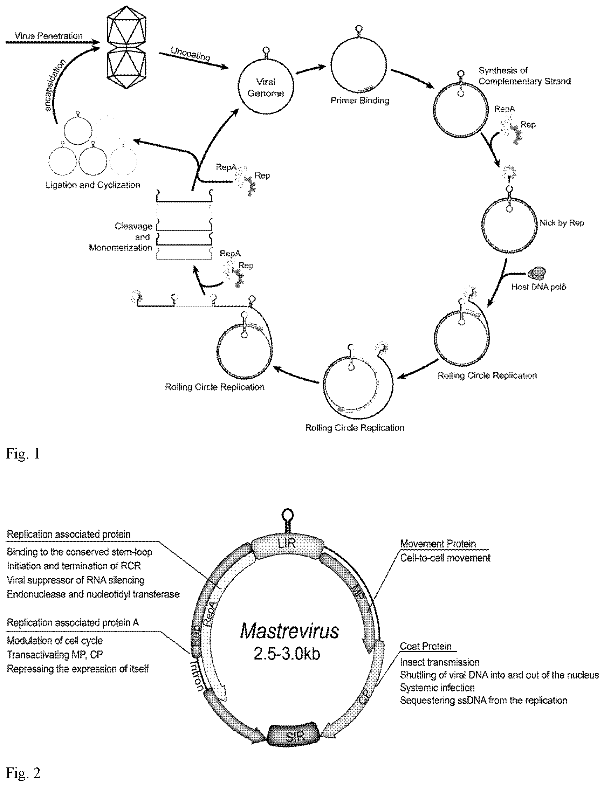

The geminivirus is the largest type of a single-stranded DNA virus in the plants, and its viral particle is of a doublet structure, a monad or a dyad, with a DNA size of 2.5-3.0 kb per molecule and a total genome size of 2.5-5.2 kb. After invading the plant cell, this family of viruses may firstly form a replication intermediate of a double-stranded DNA in the cell nucleus, and then a viral genome is amplified in a mode of rolling-circle replication under the action of a replication initiation protein Rep/RepA encoded by the viruses and an endogenous DNA polymerase in the cells (as shown in Fig. 1).

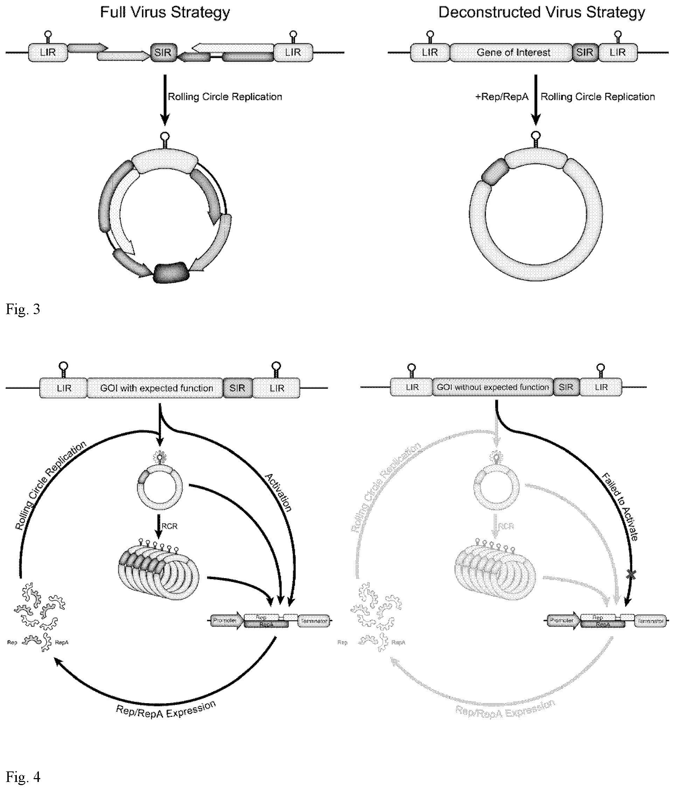

Up to now, more than 500 species are already found in Geminiviridae; and according to the genome structure, the Geminiviridae is divided into 9 genera. Only the Mastrevirus genus may infect monocotyledons, its structure is the simplest, and it is also most thoroughly researched. Members in the Mastrevirus genus are all monad viruses, its genome size is 2500 bp-2800 bp, and a total of 4 proteins are encoded, namely a mobile protein (MP), a capsid protein (CP), and the replication initiation proteins Rep and RepA (as shown in Fig. 2). Rep and RepA are encoded by a common piece of DNA, and two transcripts are obtained in a mode of alternative splicing. Rep/RepA is related to initiation and termination of the virus rolling-circle replication, inhibition of plant immunity, and regulation of virus gene expression. MP and CP are respectively related to movement and package of virions, but not to the replication. In addition, the genome of the members in the Mastrevirus genus also contains two gene spacer regions: a large intergenic region (LIR) and a small intergenic region (SIR). The former is a bidirectional promoter and contains a stable stem-loop structure, which may be recognized by Rep/RepA and is a replication starting point of the rolling-circle replication; and the latter is a bi-directional terminator and is related to the formation of a double-stranded DNA intermediate in the process of the replication. Since LIR and SIR are the only two cis-acting elements required for the virus replication, and Rep/RepA is the only trans-acting factor required, the researchers develop a deconstructed virus replicon (as shown in Fig. 3), namely it only contains LIR and SIR, and the rest portions may be any sequences, Rep/RepA may be in-situ or ectopic expressed, and drive the rolling-circle replication of the replicon.

### Brief Description of Invention

The present invention provides a method for directed evolution of a genetic element to obtain a mutant of the genetic element with a desired function, and the method includes:
i) providing a library of the mutants of the genetic element, which contains a plurality of mutants of the genetic element respectively inserted into vectors containing a geminivirus replicon, and wherein the mutant is inserted into the geminivirus replicon so that the mutant is amplified while the geminivirus replicon is replicated,
ii) transforming a population of plant cells with the library, and
iii) culturing the population of the plant cells, detecting and selecting the genetic element mutant enriched in the population of the plant cells,
wherein the replication level of the geminivirus replicon in the plant cells is configured to be associated with the desired function of the genetic element mutant.

In some embodiments, the genetic element is selected from a protein coding sequence; a functional RNA coding sequence, such as tRNA or siRNA coding sequences; and an expression regulatory sequence such as a promoter sequence, an enhancer sequence, or a terminator sequence.

In some embodiments, the genetic element is derived from a plant, or is expected to be applied in a plant.

In some embodiments, the library of the mutants of the genetic element is obtained by respectively inserting the plurality of the mutants of the genetic element into the vectors containing the geminivirus replicon.

In some embodiments, the plurality of the mutants of the genetic element are generated by random mutagenesis of the genetic element.

In some embodiments, the library is generated by performing random mutagenesis on the genetic element that is already inserted into the vector containing the geminivirus replicon.

In some embodiments, the vector containing the geminivirus replicon is a circular DNA, such as a plasmid or a minicircle DNA.

In some embodiments, the vector containing the geminivirus replicon contains at least one LIR, for example, the LIR contains a nucleotide sequence shown in SEQ ID NO: 1.

In some embodiments, the vector containing the geminivirus replicon further contains at least one SIR, for example, the SIR contains a nucleotide sequence shown in SEQ ID NO: 2.

In some embodiments, the vector containing the geminivirus replicon contains one LIR.

In some embodiments, the vector containing the geminivirus replicon contains two LIRs.

In some embodiments, in the vector containing the geminivirus replicon, the mutant of the inserted genetic element is operably linked to an expression regulatory sequence.

In some embodiments, the vector containing the geminivirus replicon further contains an expression cassette of the geminivirus Rep and/or RepA protein.

In some embodiments, the vector containing the geminivirus replicon does not contain the expression cassette of the geminivirus Rep and/or RepA protein.

In some embodiments, the method further includes introducing another vector for expressing the geminivirus Rep and/or RepA protein into the plant cell.

In some embodiments, the population of the plant cells is co-transformed with the another vector for expressing the geminivirus Rep and/or RepA protein and the library.

In some embodiments, the plant cell already contains the vector for expressing the geminivirus Rep and/or RepA protein, and/or the genome of the plant cell is already integrated with the expression cassette of the geminivirus Rep and/or RepA protein.

In some embodiments, the geminivirus Rep protein comprises an amino acid sequence shown in SEQ ID NO: 3, or comprises an amino acid sequence with amino acid substitution K229E or Y20C relative to SEQ ID NO: 3, preferably comprises an amino acid sequence shown in SEQ ID NO: 4.

In some embodiments, the geminivirus RepA protein comprises an amino acid sequence shown in SEQ ID NO: 5, or comprises an amino acid sequence with amino acid substitution K229E or Y20C relative to SEQ ID NO: 5, preferably comprises an amino acid sequence shown in SEQ ID NO: 6.

In some embodiments, wherein in the transformation of step ii), the number of vector molecules containing the mutants in the library is 10³ to 10⁵ times of the number of the cells in the population of the plant cells.

In some embodiments, wherein in step iii), the detecting and selecting the genetic element mutant enriched in the population of the plant cells may be performed by high-throughput sequencing.

In some embodiments, it further includes a step iv) identifying the function of the enriched genetic element mutant.

In some embodiments, the plant is a monocotyledon or a dicotyledon, for example, it is selected from corn, wheat, rice, barley, sorghum, kidney bean, beet, tomato, cassava, cucumber, arabidopsis and tobacco.

In some embodiments, the expression or activity of the geminivirus Rep and/or RepA protein in the plant cell is coupled with the desired function of the genetic element mutant, thereby achieving the directed evolution of the genetic element.

In some embodiments, the genetic element with the desired function activates Rep/RepA expression, thereby driving the rolling-circle replication to achieve self-enrichment; and the genetic element without the desired function cannot activate the Rep/RepA expression to achieve enrichment, thereby the directed evolution of the genetic element is achieved.

In some embodiments, the genetic element is a promoter.

In some embodiments, the method further includes placing a promoter library to be evolved upstream of Rep/RepA in the replicon.

In some embodiments, the genetic element is a cauliflower mosaic virus (CaMV) 35S promoter TATA-box.

In some embodiments, the genetic element is a sequence encoding a transcription activator.

In some embodiments, the method further includes inserting a recognition sequence of the transcription activator upstream of Rep/RepA and inserting a minimal transcription initiation element between the recognition sequence and Rep/RepA; and placing a transcription activator library to be evolved in the replicon.

In some embodiments, the genetic element is a DNA binding domain.

In some embodiments, the method further includes inserting a target binding sequence of the DNA binding domain upstream of Rep/RepA, and inserting a minimal transcription initiation element between the recognition sequence and Rep/RepA; and placing a fusion protein of the DNA binding domain to be evolved and a transcription activator without sequence specificity in the replicon.

In some embodiments, the genetic element is a sequence encoding a recombinase.

In some embodiments, the method further includes dividing Rep/RepA into two portions, and placing at two ends of the recombinase recognition sequence; and placing a sequence encoding the recombinase to be evolved in the replicon.

In some embodiments, the method further includes adding a 5' intron and a 3' intron between Rep/RepA and the recombinase recognition sequence.

In some embodiments, the genetic element is a prime editing guide RNA (pegRNA).

In some embodiments, the method further includes inserting a target site at N terminal of Rep/RepA, allowing frame-shifting of the open reading frame of Rep/RepA; and inserting an expression cassette of the pegRNA into the geminivirus replicon, and inserting a fluorescence reporter system into its two ends.

In some embodiments, the desired function of the genetic element is coupled with the expression of a nuclease.

In some embodiments, the nuclease is a sequence specific nuclease.

In some embodiments, the genetic element with the desired function activates the expression of the nuclease or guides the nuclease to cut its recognition site, thereby driving the rolling-circle replication to achieve the self-enrichment; and the genetic element without the desired function cannot allow the nuclease to cut its recognition site and the enrichment cannot be achieved, thereby the directed evolution of the genetic element is achieved.

In some embodiments, the genetic element is a DNA binding domain.

In some embodiments, the method further includes fusing a DNA binding domain library to be evolved with a non-sequence specific nuclease, and placing the same in the replicon together with its recognition sequence.

In some embodiments, the genetic element is a sequence encoding a non-sequence specific nuclease.

In some embodiments, the genetic element is a sequence encoding a transcription activator.

In some embodiments, the method further includes inserting a recognition sequence of the transcription activator upstream of the nuclease, and inserting a minimal transcription initiation element between the recognition sequence and the nuclease; and placing a transcription activator library to be evolved in the replicon together with the recognition sequence of the nuclease.

In some embodiments, the genetic element is a sequence encoding a recombinase.

In some embodiments, the method further includes placing a recombinase library to be evolved and the recognition sequence of the nuclease in the replicon; and dividing the nuclease into two portions, and placed in two ends of the recombinase recognition sequence.

In some embodiments, the method further includes adding a 5' intron and a 3' intron between the nuclease and the recombinase recognition sequence.

In some embodiments, the genetic element is a protospacer adjacent motif (PAM) of a Cas protein.

In some embodiments, the method further includes placing PAM to be evolved and a target sequence of the Cas protein together in the replicon.

In some embodiments, the genetic element is a sgRNA.

In some embodiments, the method further includes placing the sgRNA to be evolved and the target sequence of the Cas protein together in the replicon.

The present invention further provides a kit for implementing the method of the present invention.

### Brief Description of the Drawings

Fig. 1 shows a rolling-circle replication model of a geminivirus.
Fig. 2 shows a genome structure of *a Mastrevirus* genus virus.
Fig. 3 shows a deconstruction virus replicon strategy for the geminivirus.
Fig. 4 shows a basic principle of a plant in vivo directed evolution system based on a primary replicon: different alleles (mutants) of a gene of interest (GOI) are placed in a geminivirus replicon, to form a library to be screened; and a desired function of GOI is coupled with expression of Rep/RepA, namely a functional allele with the desired function may cause the expression of Rep/RepA in a plant cell, while a non-functional allele without the desired function may not cause the expression of Rep/RepA in the plant cell.
Fig. 5 shows a method of using the primary replicon directed evolution system to achieve promoter directed evolution.
Fig. 6 shows a method of using the primary replicon directed evolution system to achieve transcription activator directed evolution.
Fig. 7 shows a method of using the primary replicon directed evolution system to achieve DNA binding domain directed evolution.
Fig. 8 shows a method of using the primary replicon directed evolution system to achieve recombinase directed evolution.
Fig. 9 shows the principle of secondary replicon formation.
Fig. 10 shows a basic principle of a plant in vivo directed evolution system based on secondary replicon.
Fig. 11 shows a method of using the secondary replicon directed evolution system to achieve DNA binding domain directed evolution.
Fig. 12 shows a method of using the secondary replicon directed evolution system to achieve non-sequence specific nuclease directed evolution.
Fig. 13 shows a method of using the secondary replicon directed evolution system to achieve transcription activator directed evolution.
Fig. 14 shows a method of using the secondary replicon directed evolution system to achieve recombinase directed evolution.
Fig. 15 shows construction of the library screened in Example 1.
Fig. 16 shows a screening result of Rep Y20 while a replication enhancer is not added in Example 1.
Fig. 17 shows a screening result of Rep Y20 while the replication enhancer is added in Example 1.
Fig. 18 shows construction of the library screened in Example 2.
Fig. 19 shows a screening result in Example 2.
Fig. 20 shows an experimental principle and vector construction of Example 3.
Fig. 21 shows a screening result in Example 3.
Fig. 22 shows construction of the library screened in Example 4.
Fig. 23 shows a screening principle of the PAM library in Example 4.
Fig. 24 shows screening results of 3 bases at a 3' end of a PAM library sequence in Example 4.
Fig. 25 shows a sequence identification diagram of 6 bases in the PAM library sequence in Example 4.
Fig. 26 shows an experimental principle and vector construction of Example 5.
Fig. 27 shows a screening result in Example 5.

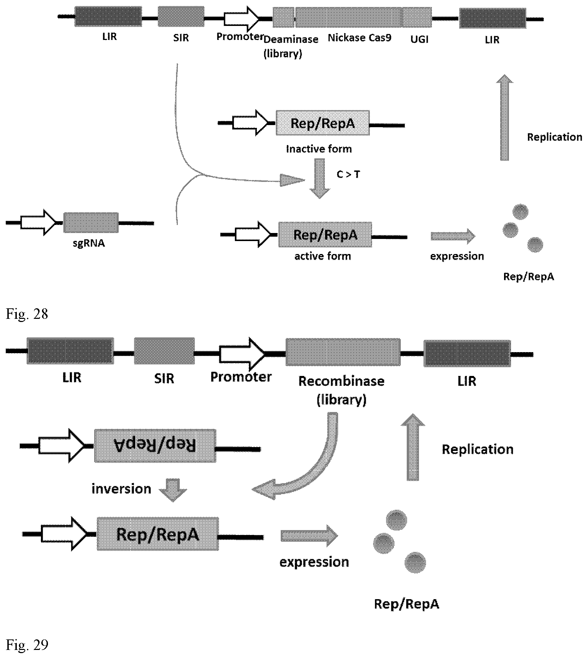

Fig. 28 shows a schematic diagram of a directed evolution principle of a base editor, wherein the plant cell is co-transformed by a base editor mutant expression library, an inactivated Rep/RepA expression vector, and a sgRNA expression construct targeting the inactivated Rep/RepA coding sequence. While a base editor mutant has the desired base editing activity, it may correct the inactivated Rep/RepA to the activated Rep/RepA, so that enrichment is obtained.
Fig. 29 shows a schematic diagram of a directed evolution principle of a recombinase, wherein the plant cell is co-transformed with a recombinase mutant expression library, and a Rep/RepA gene and promoter reverse expression vector. While a recombinase mutant has the desired activity, it may invert a reverse Rep/RepA gene (inversion), so that it may be driven and expressed by a promoter to achieve enrichment of the recombinase mutant.

### Detailed Description of the Invention

In one aspect, the present invention provides a method for directed evolution of a genetic element to obtain a mutant of the genetic element with a desired function, and the method includes:
i) providing a library of the mutant of the genetic element, which contains a plurality of mutants of the genetic element respectively inserted into a vector containing a geminivirus replicon, and wherein the mutant is inserted into the geminivirus replicon so that the mutant is amplified while the geminivirus replicon is replicated,
ii) transforming a population of plant cells with the library, and
iii) culturing the population of the plant cells, detecting and selecting the genetic element mutant enriched in the population of the plant cells,
wherein the replication level of the geminivirus replicon in the plant cell is configured to be associated with the desired function of the genetic element mutant.

As used wherein, the term "genetic element" refers to a nucleotide sequence/nucleic acid molecule that may achieve a specific function in a cell, preferably in a plant cell. Examples of the genetic element include, but are not limited to, a protein coding sequence, a functional RNA (such as tRNA and siRNA) coding sequence, and an expression regulatory sequence such as a promoter sequence, an enhancer sequence, or a terminator sequence. In some preferred embodiments, the genetic element is derived from a plant, or is expected to be applied in a plant.

In the context of this description, the term "library" is used with its known meaning in the field of cell biology and molecular biology, and it refers to a collection of different nucleic acid fragments/nucleic acid molecules. A specific type of the library is a library containing random mutants generated by random mutagenesis. Another example is a designed (or synthesized) library, and it contains different specially engineered nucleic acid fragments/nucleic acid molecules.

In some embodiments, the library of mutants of the genetic element is obtained by respectively inserting a plurality of the mutants of the genetic element into a vector containing the geminivirus replicon. In some embodiments, the plurality of the mutants of the genetic element is generated by the random mutagenesis.

In some embodiments, the library may be generated by performing random mutagenesis on the genetic element that has been inserted into the vector containing the geminivirus replicon.

In the context of this description, the term "random mutagenesis" is used with its meaning known in the field of cell biology and molecular biology; and it refers to a method in which a DNA mutation is introduced randomly to generate mutant genes and proteins. Then, many of these mutant genes may be compiled into the library. Non-limiting examples of the random mutagenesis method are error prone polymerase chain reaction (PCR), ultraviolet (UV) radiation and chemical mutagen.

"Geminivirus" is a DNA virus that infects plants, and it is a virus having 1 or 2 single-stranded circular DNA molecules. Examples of the geminivirus include, but are not limited to: maize streak virus (MSV), wheat dwarf virus (WDV), bean yellow dwarf virus (BeYDV) and other viruses belonging to Mastrevirus genus, beet curytop virus (BCTV) and other viruses belonging to beet curytop virus genus, tomato pseudo-curytop virus (TPCTV) and other viruses belonging to tomato pseudo-curytop virus genus, as well as bean golden mosaic virus (BGMV), African cassava mosaic virus (ACMV), squash leaf curl virus (SLCV), tomato golden mosaic virus (TGMV), and tomato yellow leaf curl virus (TYLCV) and the like. In some preferred embodiments, the geminivirus is WDV

The plant of the present invention may be a monocotyledon or a dicotyledon, as long as the geminivirus replicon may be replicated in its cell. The suitable plants include, but are not limited to, corn, wheat, rice, barley, sorghum, kidney bean, beet, tomato, cassava, cucumber, arabidopsis, tobacco and the like.

In some embodiments, the plant cell is an isolated plant cell. In some preferred embodiments, the plant cell is a protoplast cell.

In some embodiments, the plant cell is a cell in a plant tissue or a plant organ or a plant body, namely the cell is not isolated from the plant tissue or the plant organ or the plant body. For example, the plant cell may be a cell in a leaf.

The "replication level" of the geminivirus replicon may be determined by detecting the copy number of the geminivirus replicon. Methods for detecting the copy number of the geminivirus replicon are known in the art, including but not limited to PCR (such as fluorescence quantitative PCR) method or sequencing (such as deep sequencing) method.

In some embodiments, the vector containing the geminivirus replicon is a circular DNA, such as a double-stranded or single-stranded circular DNA. In some embodiments, the vector containing the geminivirus replicon is a plasmid. In some embodiments, the vector containing the geminivirus replicon is a minicircle DNA.

In some embodiments, the vector containing the geminivirus replicon contains at least one LIR.

In some embodiments, the vector containing the geminivirus replicon further contains at least one, such as one SIR.

In some embodiments, the vector containing the geminivirus replicon contains one LIR. In this case, the entire vector is replicated as the geminivirus replicon replicates.

In some preferred embodiments, the vector containing the geminivirus replicon contains two LIRs. In some embodiments, one SIR is contained between the two LIRs. In this case, the sequence from the first LIR to the second LIR (containing SIR) is replicated as the geminivirus replicon replicates. Preferably, the genetic element mutant is located between the two LIRs.

In some embodiments, the LIR comprises a nucleotide sequence shown in SEQ ID NO:1. In some embodiments, the SIR comprises a nucleotide sequence shown in SEQ ID NO: 2.

In some embodiments, in the vector containing the geminivirus replicon, the inserted mutant of the genetic element is operably linked to an expression regulatory sequence.

The "expression regulatory sequence" and "expression regulatory element" may be interchangeably used, and refer to nucleotide sequences that are located at upstream (5' non-coding sequence), middle or downstream (3' non-coding sequence) of the coding sequence, and affect the transcription, RNA processing or stability or translation of the relevant coding sequence. The plant expression regulatory element refers to a nucleotide sequence that may control the transcription, RNA processing or stability or translation of the interested nucleotide sequence in a plant. The expression regulatory sequence may include, but not limited to, a promoter, a translation leader sequence, an intron, and a polyadenylation recognition sequence. The "promoter" refers to a nucleic acid fragment that may control the transcription of another nucleic acid fragment. In some embodiments of the present invention, the promoter is a promoter that may control gene transcription in the plant cell, regardless of whether it is derived from the plant cell. The promoter may be a constitutive promoter or a tissue specific promoter or a developmental regulatory promoter or an inducible promoter.

In some embodiments, the vector containing the geminivirus replicon also contains an expression cassette of a geminivirus Rep and/or RepA protein.

The expression cassette of the geminivirus Rep and/or RepA protein usually contains a coding nucleotide sequence of the geminivirus Rep and/or RepA protein and a expression regulatory element operably linked thereto.

In some embodiments, the vector containing the geminivirus replicon does not contain the expression cassette of the geminivirus Rep and/or RepA protein. Therefore, the geminivirus Rep and/or RepA protein needs to be provided in a trans manner.

In some embodiments, the method further includes introducing another vector for expressing the geminivirus Rep and/or RepA protein into the plant cell. The vector for expressing the geminivirus Rep and/or RepA protein usually contains the expression cassette of the geminivirus Rep and/or RepA protein. In some embodiments, the population of the plant cells is co-transformed with the another vector for expressing the geminivirus Rep and/or RepA protein and the library.

In some embodiments, the plant cell already contains the vector for expressing the geminivirus Rep and/or RepA protein, and/or the genome of the plant cell is already integrated with the expression cassette of the geminivirus Rep and/or RepA protein.

In some embodiments, the geminivirus Rep protein comprises an amino acid sequence shown in SEQ ID NO: 3, or comprises an amino acid sequence with amino acid substitution K229E or Y20C relative to SEQ ID NO: 3, for example SEQ ID NO: 4. In some embodiments, the geminivirus RepA protein comprises an amino acid sequence shown in SEQ ID NO: 5, or comprises an amino acid sequence with amino acid substitution K229E or Y20C relative to SEQ ID NO: 5, for example SEQ ID NO: 6. In some preferred embodiments, the geminivirus Rep protein comprises an amino acid sequence shown in SEQ ID NO: 4. In some preferred embodiments, the geminivirus RepA protein comprises an amino acid sequence shown in SEQ ID NO: 6.

"The replication level of the geminivirus replicon in the plant cell is configured to be associated with the desired function of the genetic element mutant" means that the replication level of the geminivirus replicon of the genetic element mutant having the desired function in the plant cell is higher than, preferably, significantly higher than the replication level of the geminivirus replicon of the genetic element mutant without the desired function in the plant cell. For example, it is possible to configure that the genetic element mutant having the desired function causes the replication of the geminivirus replicon in the plant cell, while the genetic element mutant without the desired function does not cause the replication of the geminivirus replicon in the plant cell; or preferably, the genetic element mutant having the desired function causes high level replication of the geminivirus replicon in the plant cell, while the genetic element mutant without the desired function causes low level replication or non-replication of the geminivirus replicon in the plant cell. While the geminivirus replicon containing the genetic element mutant having the desired function is amplified or significantly amplified, due to the replication or the high level replication, compared with other geminivirus replicons without the genetic element mutant having the desired function, the enrichment of the genetic element mutant having the desired function may be achieved.

The Rep and/or RepA protein is a replication initiation protein of the geminivirus, and its activity or expression level is usually positively correlated with the replication level (such as the copy number) of the geminivirus within a certain range. Therefore, in some embodiments, "the activity or expression level of the geminivirus Rep and/or RepA protein in the plant cell may be configured to be associated with the desired function of the genetic element mutant". For example, the activity or expression level of the geminivirus Rep and/or RepA protein in the plant cell containing the genetic element mutant having the desired function may be higher than, preferably, significantly higher than the activity or expression level of the geminivirus Rep and/or RepA protein in the plant cell containing the genetic element mutant without the desired function. In some embodiments, the activity of the geminivirus Rep and/or RepA protein is the activity of mediating (initiating) the replication of the geminivirus replicon, and it may be determined, for example, by detecting the replication level of the geminivirus replicon. For example, the genetic element mutant having the desired function may cause the expression of the geminivirus Rep and/or RepA protein in the plant cell, while the genetic element mutant without the desired function causes no expression of the geminivirus Rep and/or RepA protein in the plant cell; or the genetic element mutant having the desired function may cause the high level expression of the geminivirus Rep and/or RepA protein in the plant cell, while the genetic element mutant without the desired function causes low level expression or no expression of the geminivirus Rep and/or RepA protein in the plant cell. Alternatively, the genetic element mutant having the desired function may cause the geminivirus Rep and/or RepA protein to be active in the plant cell, while the genetic element mutant without the desired function causes the geminivirus Rep and/or RepA protein to be inactive in the plant cell; or the genetic element mutant having the desired function may cause the high activity of the geminivirus Rep and/or RepA protein in the plant cell, while the genetic element mutant without the desired function causes low activity or no activity of the geminivirus Rep and/or RepA protein in the plant cell. The expression or activity or high level expression or high activity of the geminivirus Rep and/or RepA protein in the plant cell may cause the amplification or significant amplification of the geminivirus replicon, thereby the enrichment of the genetic element mutant having the desired function is achieved.

The "low level" or "low activity" mentioned herein is relative to the "high level" or "high activity", which does not necessarily mean that it is lower than the normal level or normal activity.

The replication level of the geminivirus in the plant cell or the activity or expression level of the Rep and/or RepA protein of the geminivirus in the plant cell may be configured as directly or indirectly associated with the desired function of the genetic element mutant. Those skilled in the art may achieve such association according to the type of a genetic element and the desired specific function of the mutant.

In the context of this description, the term "expression level" is used with its meaning known in the field of cell biology and molecular biology; and it refers to the transcription level and/or translation level of a DNA fragment and its derived mRNA respectively.

For example, while the genetic element is a expression regulatory element (such as the promoter and the enhancer), the coding sequence of the geminivirus Rep and/or RepA protein may be directly placed under the control of the expression regulatory element mutant (such as a promoter mutant and an enhancer mutant). If the mutant can enhance gene expression, it may cause increased expression of the Rep and/or RepA protein, and the increased expression of the Rep and/or RepA protein may cause increased replication of the geminivirus replicon and the corresponding expression regulatory element mutant (such as the promoter mutant) in turn. By detecting the significantly enriched mutant sequence, the expression regulatory element mutant that enhances gene expression, namely an evolved expression regulatory element, can be obtained.

While the genetic element is a protein coding sequence, the desired function of the protein encoded by it may be associated with the activity or expression level of the geminivirus Rep and/or RepA protein.

For example, if the directed evolution needs to be performed on a base editor, the plant cell may be co-transformed with a base editor mutant library constructed in a vector containing the geminivirus replicon, an expression vector containing the coding sequence of an inactivated Rep/RepA protein specifically designed for the desired function of the base editor, and a sgRNA expression construct targeting the coding sequence of the inactivated Rep/RepA. While the base editor mutant in the plant cell has the desired base editing activity, it may correct the specifically designed inactivated Rep/RepA to the activated Rep/RepA, thereby inducing the replication of the geminivirus replicon, so that the mutant may be enriched.

Alternatively, if the directed evolution needs to be performed on a recombinase, the plant cell may be co-transformed with a library of the recombinase mutants constructed in a vector containing the geminivirus replicon, and an expression vector in which Rep/RepA coding sequence and promoter are reversely arranged. While the recombinase mutant in the plant cell has the desired activity, it may invert the reversed Rep/RepA gene (inversion), thereby the Rep/RepA gene may be driven and expressed by the promoter, and the replication of the geminivirus replicon is induced to achieve enrichment of the recombinase mutant.

In some embodiments, in the transformation of step ii), the number of vector molecules containing the mutants in the library is 10³ to 10⁵ times of the number of the cells in the population of the plant cells. This ratio may reduce the probability of multiple different vector molecules transformed into a same cell and reduce the background of screening while the transformation efficiency is guaranteed.

In the context of this description, the term "primary replicon" refers to a replicon formed by the recognition and cyclization of LIRs tandem on the vector by Rep/RepA in a plant geminivirus system. The primary replicon is amplified by rolling-circle replication.

In some embodiments, the directed evolution of the genetic element is accomplished by as follows: the expression or activity of the geminivirus Rep and/or RepA protein in the plant cell is coupled with the desired function of the genetic element mutant. In some embodiments, the directed evolution of the genetic element is accomplished by as follows: the genetic element having the desired function activates Rep/RepA expression, thereby the rolling-circle replication is driven, to achieve self enrichment; and the genetic element without the function may not activate the Rep/RepA expression, so the enrichment cannot be achieved.

In some embodiments, the genetic element is a promoter. In some embodiments, the method i) further includes placing a promoter library to be evolved upstream of Rep/RepA in the replicon. The promoter having the function may drive the expression of downstream Rep/RepA, drive its own rolling-circle replication, increase the copy number, and achieve the self enrichment; and the promoter without the function may not drive the expression of the downstream Rep/RepA, and may not achieve the enrichment, thereby the directed evolution of the promoter is achieved.

In some embodiments, the genetic element is a CaMV 35S promoter TATA-box.

In the context of this description, the term "transcription activator" refers to a DNA binding protein that may activate gene expression. The transcription activator binds to an upstream promoter element to regulate the transcription process.

In some embodiments, the genetic element is a sequence encoding the transcription activator. In some embodiments, the method i) further includes inserting a recognition sequence of the transcription activator into the upstream of Rep/RepA, and inserting a minimal promoter between the recognition sequence and Rep/RepA; and placing a transcription activator library to be evolved in the replicon.

In some embodiments, the genetic element is a DNA binding domain. In some embodiments, the method i) further includes inserting a target binding sequence of the DNA binding domain to the upstream of Rep/RepA, and inserting a minimal transcription initiation element between the target binding sequence and Rep/RepA; and placing a fusion protein formed by the DNA binding domain to be evolved and the transcription activator without sequence specificity in the replicon together. The transcription activator having the desired function may bind to its recognition sequence, and activate the expression of the downstream Rep/RepA, thereby the rolling-circle replication is driven to achieve the self-enrichment; and the transcription activator without the function cannot activate the downstream Rep/RepA, and cannot achieve the enrichment, thereby the directed evolution of the transcription activator is achieved.

In the context of this description, the term "recombinase" refers to an enzyme involved in the process of gene directed recombination. It is responsible for identifying and cutting a specific recombination site, and linking two molecules involved in recombination. In some embodiments, the genetic element is a sequence encoding the recombinase. In some embodiments, the method i) further includes dividing Rep/RepA into two portions, and placing to two ends of a recombinase recognition sequence; and placing a sequence encoding the recombinase to be evolved in the replicon. In some embodiments, the method i) further includes adding a 5' intron and a 3' intron between Rep/RepA and the recombinase recognition sequence. The recombinase with the desired function may recognize its specific recognition site, mediate the DNA recombination, and normally express Rep/RepA, thereby the rolling-circle replication is driven to achieve self enrichment; and the recombinase without the desired function cannot mediate the DNA recombination, cannot express Rep/RepA, and cannot achieve the enrichment, thereby the directed evolution of the recombinase is achieved.

In some embodiments, the genetic element is a prime editing guide pegRNA. In some embodiments, the method i) further includes inserting a target site to N terminal of Rep/RepA to allow frame-shifting of the open reading frame of Rep/RepA; and inserting an expression cassette of pegRNA into the geminivirus replicon, and inserting fluorescence reporter systems into its two ends. If the virus replicon generates the rolling-circle replication under the action of the pegRNA, a fluorescence signal is reported; and if the virus replicon does not generate the rolling-circle replication, there is no fluorescence signal. In some embodiments, while a tobacco leaf is transformed with a low concentration library, active pegRNA is significantly enriched, because the low concentration may guarantee that only one vector enter the cell for most cells, and the screening requirements are satisfied.

In the context of this description, the term "nuclease" refers to a type of enzymes that catalyze hydrolysis of phosphate diester bond when using a nucleic acid as a substrate. In some embodiments, the desired function of the genetic element is coupled with nuclease expression. In some embodiments, the nuclease is a sequence specific nuclease.

In the context of this description, the term "secondary replicon" refers to a replicon formed as follows: the primary replicon of the geminivirus generates a double-stranded DNA break (DSB) under the action of the sequence specific nuclease, and the break may be linked with a right border (RB) of the plasmid under the guidance of VirD2, thereby a replicon is formed under the action of Rep/RepA. In some embodiments, the directed evolution of the genetic element is accomplished by as follows: the genetic element with the desired function activates the expression of the nuclease or guides the nuclease to cut its recognition site, thereby the secondary replicon is formed, and the rolling-circle replication is driven to achieve the self enrichment; and the genetic element without the function may not allow the nuclease to cut its recognition site, thereby the secondary replicon cannot be formed, and the enrichment cannot be achieved.

In some embodiments, the genetic element is a DNA binding domain. In some embodiments, the method i) further includes fusing a DNA binding domain library to be evolved with a non-sequence specific nuclease, and placing in the replicon together with the recognition sequence thereof. The DNA binding domain with the desired function may guide the nuclease to cut the target sequence, and generate a secondary replicon under the action of virD2; and the DNA binding domain without the desired function cannot guide the nuclease to cut the target sequence, thereby the secondary replicon cannot be generated. By detecting the secondary replicon, the directed evolution of the DNA binding domain is achieved.

In some embodiments, the genetic element is a sequence encoding a non-sequence specific nuclease.

In some embodiments, the genetic element is a sequence encoding a transcription activator. In some embodiments, the method i) further includes inserting a recognition sequence of the transcription activator to upstream of the nuclease, and inserting a minimal transcription initiation element between the recognition sequence and the nuclease; and placing a transcription activator library to be evolved in the replicon together with the recognition sequence of the nuclease. The transcription activator with the desired function may activate the expression of the nuclease, which in turn cuts its recognition sequence, and the secondary replicon is formed under the action of virD2; and the transcription activator without the desired function cannot activate the expression of the nuclease, and thereby the secondary replicon cannot be generated. By detecting the secondary replicon, the directed evolution of the transcription activator may be achieved.

In some embodiments, the genetic element is a sequence encoding a recombinase. In some embodiments, the method i) further includes placing a recombinase library to be evolved and a recognition sequence of a nuclease in the replicon; and dividing the nuclease into two portions which are placed at two ends of a recognition sequence of the recombinase. In some embodiments, the method i) further includes adding a 5' intron and a 3' intron between the nuclease and the recombinase recognition sequence. The recombinase with the desired function may mediate the DNA recombination to express the nuclease, which in turn cuts its recognition site to generate a secondary replicon; and the recombinase without the desired function cannot mediate the DNA recombination, the nuclease cannot be expressed normally, and the secondary replicon cannot be generated. By detecting the secondary replicon, the directed evolution of the recombinase may be achieved.

In some embodiments, the genetic element is PAM of a Cas protein. In some embodiments, the method further includes placing PAM to be evolved and a target sequence of the Cas protein in the replicon together. PAM that may be recognized by the Cas may generate DSB in the target region to form a secondary replicon, and information of PAM may be preserved in the secondary replicon; and PAM that cannot be recognized by Cas cannot generate a DSB, and the secondary replicon cannot be formed. By detecting the secondary replicon, the directed evolution of PAM may be achieved.

In some embodiments, the genetic element is a sgRNA. In some embodiments, the method further includes placing sgRNA to be evolved and the target sequence of a Cas protein in the replicon together. sgRNA with the desired activity can guide Cas to cut a target site located at its downstream so as to form a secondary replicon; and sgRNA without the activity cannot generate a DSB, so the secondary replicon cannot be generated. By detecting the secondary replicon, the directed evolution of sgRNA may be achieved.

In some embodiments, in the step iii), the detecting and selecting of the genetic element mutants enriched in the population of the plant cells may be performed by high-throughput sequencing. For example, the total DNA of the population of the plant cells may be extracted, and high-throughput sequencing may be performed for the genetic element.

In some embodiments, the method further includes a step iv) of identifying the function of the enriched genetic element mutant.

In one aspect, the present invention provides a genetic element mutant or a coding product thereof obtained by the method of the present invention, and the use of the obtained genetic element mutant or the coding product thereof in plants, especially in plant genetic engineering.

In one aspect, the present invention provides a kit for implementing the method of the present invention. The kit may include, for example, a vector containing the geminivirus replicon, and/or a vector for expressing the geminivirus Rep and/or RepA protein. The kit may further include a specification for implementing the method of the present invention.

### Examples

Further understanding of the present invention may be obtained by referring to some specific embodiments given herein, and these embodiments are only used to describe the present invention, but are not intended to limit the scope of the present invention. Apparently, various modifications and changes may be made to the present invention without departing from the essence of the present invention. Therefore, these modifications and changes are also within a scope of protection claimed in the present application.

WDV and BeYDV replication subsystems are developed. The two viruses belong to the Mastrevirus genus, the genome structures are very similar, and they may achieve the high-efficient genome amplification in monocotyledons and dicotyledons respectively.

### 1. Directed evolution system based on primary replicon

At present, there are many directed evolution systems with various methods, but the core idea is much the same, namely GOI with a function is enriched, and GOI without the function is filtered out. However, compared with bacteria and yeast, the speed of plant cell division (namely replication of genomic DNA) is very slow, and it is difficult to meet the needs. Therefore, it is expected to use virus replication to replace the plant cell division so as to achieve the enrichment of GOI.

In a plant geminivirus system, LIRs that exists tandemly on a vector may be recognized by Rep/RepA, and cyclized into a primary replicon (PR), and then rolling-circle replication is performed, so the copy number may be increased by about 3 orders of magnitude. Herein, Rep/RepA is the only protein required for this process. Based on this principle, a screening library of GOI (it may be generated by error prone PCR or saturation mutation) may be cloned into the geminivirus replicon, and supplemented by other elements, so that a desired function of GOI is coupled with the expression of Rep/RepA, and thus a plant in vivo directed evolution system based on the primary replicon of the geminivirus is constructed (as shown in Fig. 4). In this system, a target gene allele with the desired function may directly or indirectly drive the expression of Rep/RepA, thereby it is enriched by itself; and the allele without the desired function may not start the expression of Rep/RepA, and it may not be enriched by itself. Then, by deep sequencing, it may be inferred which allele has the function, thereby the purpose of evolution is achieved.

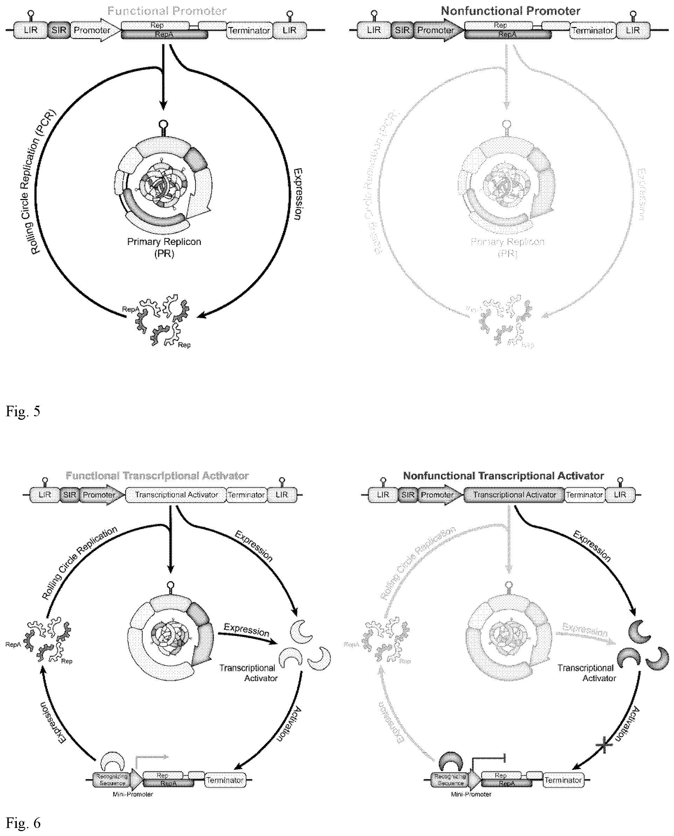

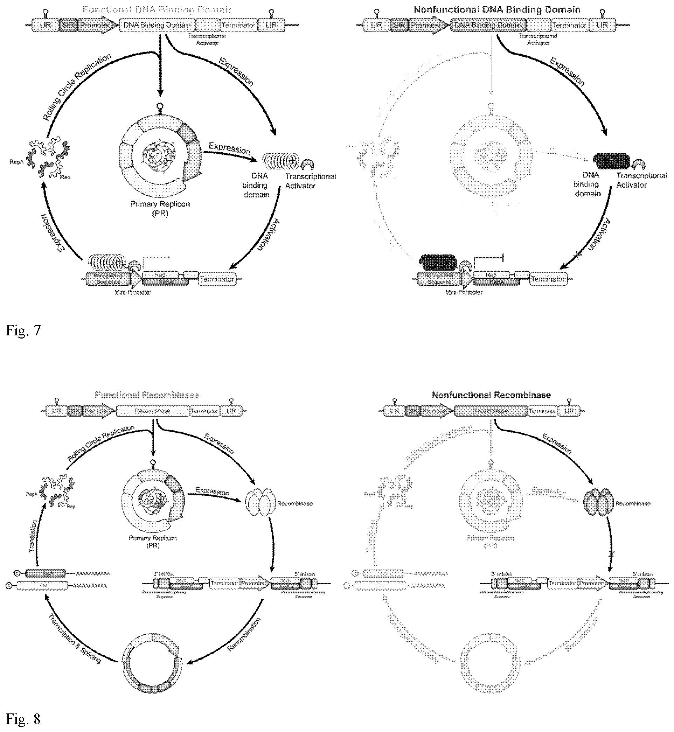

By using the primary replicon, the inventor expects that the directed evolution of the genetic element such as a promoter, a transcription activator, a DNA binding protein, and a recombinase may be achieved. In order to achieve the directed evolution of a promoter, the promoter library to be evolved may be placed to upstream of Rep/RepA. The promoter with function may drive the expression of downstream Rep/RepA, drive the own rolling-circle replication, increase the copy number, and achieve the enrichment; and the promoter without function cannot drive the expression of the downstream Rep/RepA, and cannot achieve the enrichment, thereby the directed evolution of the promoter is achieved (as shown in Fig. 5). In the directed evolution of a transcription activator, the recognition sequence of the transcription activator may be inserted to upstream of Rep/RepA, and supplemented by a minimal transcription initiation element; and a transcription activator library to be evolved is inserted into the replicon. The transcription activator with function may bind to its recognition sequence, and activate the expression of the downstream Rep/RepA, thereby the rolling-circle replication is driven to achieve the self enrichment; and the transcription activator without function may not activate the downstream Rep/RepA, and may not achieve the enrichment, thereby the directed evolution of the transcription activator is achieved (as shown in Fig. 6). In the directed evolution of a DNA binding domain, the DNA binding domain to be evolved and a transcription activator without sequence specificity may be combined to form a fusion protein, and placed in the replicon together; and the target binding sequence of the DNA binding domain is inserted to upstream of Rep/RepA, and supplemented by a mini-promoter. In this way, the DNA binding domain having function may bind to its target sequence, and bring the transcription activator to the mini-promoter, the expression of the downstream Rep/RepA is driven, and the rolling-circle replication is driven to achieve the self enrichment; and the DNA binding domain without function cannot bind to the target sequence, and the downstream Rep/RepA cannot be activated, so that the enrichment cannot be achieved, thereby the directed evolution of the DNA binding domain is achieved (as shown in Fig. 7). In order to achieve the directed evolution of a recombinase, the recombinase to be evolved may be placed in the replicon; and Rep/RepA is divided into two portions and placed to two ends of the recombinase recognition sequence. In order to guarantee that Rep/RepA may function normally after recombination, a 5' intron and a 3' intron may be added between Rep/RepA and the recombinase recognition sequence, so that the recombinase recognition sequence may be cut off after transcription, and Rep/RepA is translated normally. In this way, the recombinase with function may recognize its specific recognition site, mediate the DNA recombination, and normally express Rep/RepA, thereby the rolling-circle replication is driven to achieve self enrichment; and the recombinase without function cannot mediate the DNA recombination, cannot express Rep/RepA, and cannot achieve the enrichment, thereby the directed evolution of the recombinase is achieved (as shown in Fig. 8).

### 2. Directed evolution system based on secondary replicon

In a plant genetic transformation system mediated by Agrobacterium tumefaciens, a series of Vir proteins encoded by the Agrobacterium tumefaciens may recognize a RB sequence on a Ti plasmid, and generate a single-strand DNA break nick at a specific position on it. Then, the VirD2 protein may covalently bind to the 5' DNA end of the nick, release a T-DNA sequence which is transferred to a plant cell nucleus. In the cell nucleus, VirD2 may recognize a DSB spontaneously generated on the plant genome, and link the T-DNA sequence to it by non homologous end joining (NHEJ) and other modes under the action of a series of host factors, so as to insert the T-DNA sequence into the plant genome.

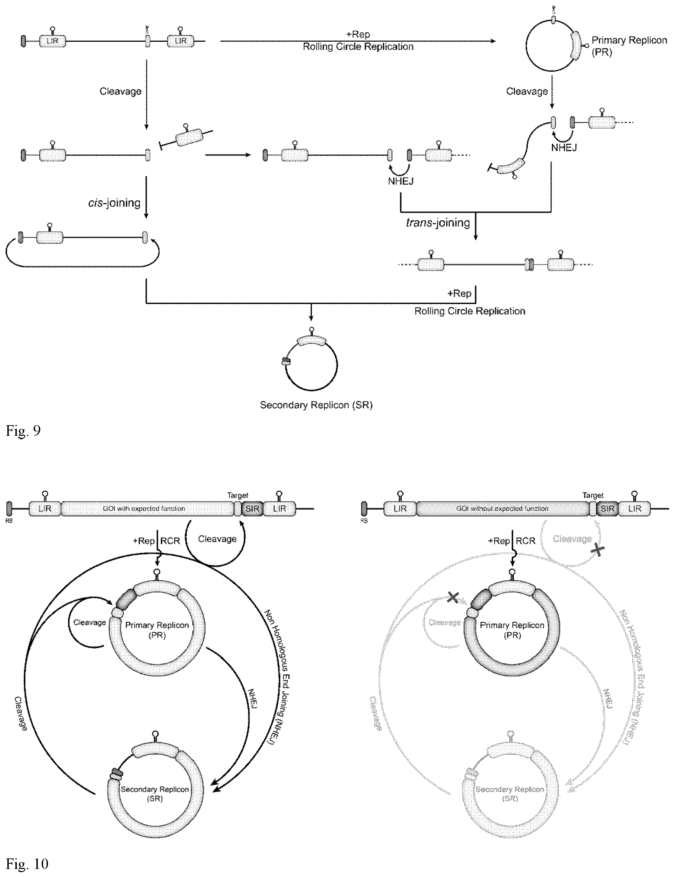

Based on this principle, it is found by the inventor from experiments that if one DSB is artificially generated in the geminivirus replicon by using a sequence specific nuclease, the RB region may be linked with the break under the guidance of VirD2, and then a secondary replicon (SR) may be formed under the action of Rep/RepA (as shown in Fig. 9). The inventors subsequently find that this linkage may be divided into two modes, cis-linkage and trans-linkage, and the former is dominant. In general, whether a secondary replicon is generated depends entirely on whether the sequence specific nuclease generates a DSB in a specific position.

In addition, compared with other directed evolution methods, the directed evolution relying on the secondary replicon also has the following advantages: first, in order to guarantee that most cells with only one vector molecule in the evolution process, the concentration of the target gene library must be kept very low, but this also means that the initial expression of GOI is very low, and it may not be enough to meet the screening requirements. In the directed evolution depending on the secondary replicon, GOI may firstly generate a first round of the rolling-circle replication under the action of Rep to form the primary replicon, and in this process, the copy number of GOI may be increased by three orders of magnitude in a short time period, and the expression is greatly increased, so that it would be enough to meet the next screening step. Second, in the process of the directed evolution, the secondary replicon allows for enriching twice: firstly, the secondary replicon is generated under the action of the sequence specific nuclease; and secondly, under the action of Rep/RepA, the secondary replicon generates the second round of the rolling-circle replication, and the copy number is greatly increased. All these make the directed evolution system relying on the secondary replicon have extraordinary advantages.

According to this principle, the secondary replicon may be used to screen or evolve sequence specific nucleases in the plants with high throughput (as shown in Fig. 10), and may also be used to research a cutting mode of the sequence specific nuclease (such as PAM of a Cas nuclease) and guide RNA (such as sgRNA of Cas9 or crRNA of Cas12a). In addition, the genetic element that may be coupled with nuclease expression may be evolved with high throughput.

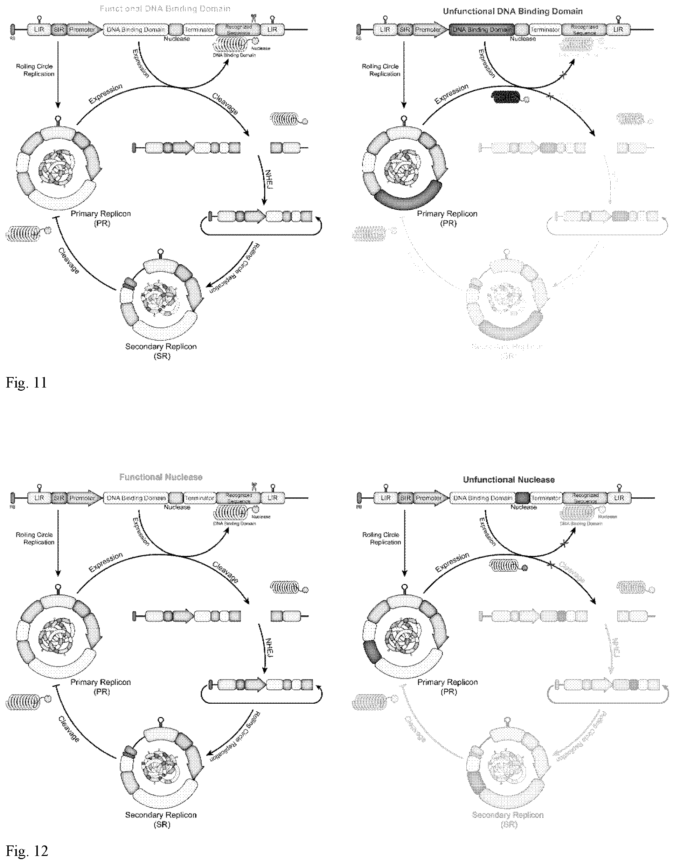

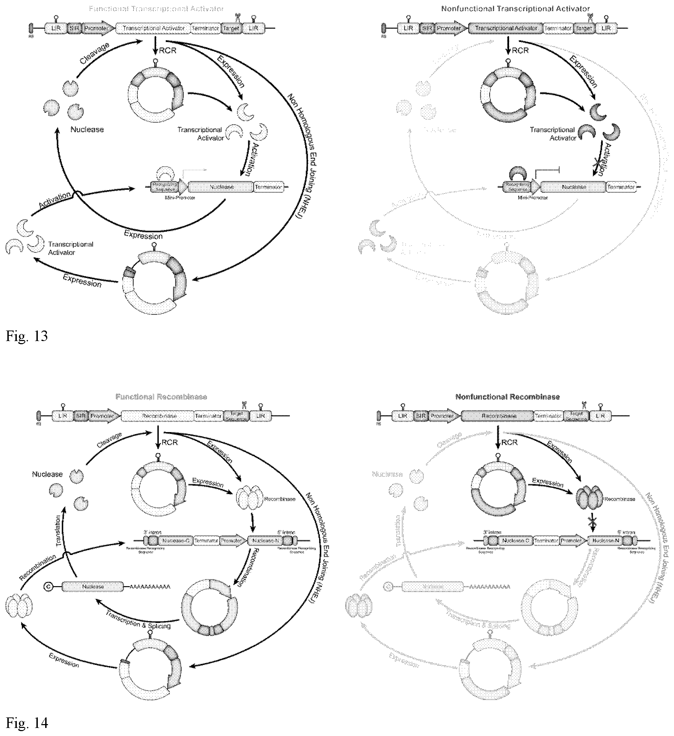

For example, a DNA binding domain may be evolved. A DNA binding domain library to be evolved is fused with a non-sequence specific nuclease and placed in the replicon together with target sequence thereof. In this way, the DNA binding domain with function may guide the nuclease to cut the target sequence and generate the secondary replicon under the action of virD2; and the DNA binding domain without function cannot guide the nuclease to cut the target sequence, thereby the secondary replicon cannot be generated. By detecting the secondary replicon, the directed evolution of the DNA binding domain is achieved (as shown in Fig. 11). Similarly, the directed evolution of the non-sequence specific nuclease may also be achieved (as shown in Fig. 12). In addition, the secondary replicon may also be used to achieve the directed evolution of a transcription activator. The recognition sequence of the transcription activator may be placed upstream of the nuclease, and supplemented by a minimal transcription initiation element (mini-promoter); and a transcription activator library to be evolved is placed in the replicon together with the recognition sequence of the nuclease. In this way, the transcription activator with function may activate the expression of the nuclease, and cut its recognition sequence, and the secondary replicon is formed under the action of virD2; and the transcription activator without function cannot activate the expression of the nuclease, and thereby the secondary replicon cannot be generated. By detecting the secondary replicon, the directed evolution of the transcription activator may be achieved (as shown in Fig. 13). The directed evolution of a recombinase may also be performed by using the directed evolution system relying on the secondary replicon. A recombinase library to be evolved and the recognition sequence of the nuclease are placed in the replicon together, and the nuclease is divided into two portions and placed on two ends of a recognition sequence of the recombinase. In order to guarantee that the nuclease can function normally after recombination, a 5' intron and a 3' intron may be added between the nuclease and the recombinase recognition sequence, so that the recombinase recognition sequence may be cut off after transcription and the nuclease is translated normally. In this way, the recombinase with function may mediate the DNA recombination to express the nuclease which will cut its recognition site to generate the secondary replicon; and the recombinase without function cannot mediate the DNA recombination, the nuclease cannot be expressed normally, and the secondary replicon cannot be generated. By detecting the secondary replicon, the directed evolution of the recombinase may be achieved (as shown in Fig. 14).

### Experimental materials and methods

### 1. Cultivation of wheat seedlings:

Wheat seeds were planted in a culture room, and cultured under the conditions of 25 + 2 °C of a temperature, 1000 Lx of an illuminance and 14~16 h/d of light, and the culture time was about 1-2 weeks.

### 2. Protoplast separation:

1) A young leaf of wheat was taken, its middle portion was cut into 0.5-1 mm of silks, put into 0.6 M of Mannitol solution and treated in the dark for 10 minutes, then filtered with a filter screen, and put into 50 ml of enzymolysis solution and digested at 20-25 °C in the dark for 5 hours (firstly static enzymolysis was performed for 0.5 h, then slowly shaken at 10 rmp for 4.5 h).
2) 10 ml of W5 solution with a pH value of 5.7 was added to dilute the enzymatic hydrolysate, and the enzymatic hydrolysate was filtered with a 75 um nylon filter membrane into a 50 ml round bottom centrifuge tube.
3) Centrifuged at 100 g at 23°C for 3 min, and the supernatant was discarded.
4) The precipitate was gently suspended with 10 ml of the W5 solution, placed on ice for 30 min so that the protoplast was gradually settled, and the supernatant was discarded.
5) An appropriate amount of monoclonal gamma globulin (MMG) solution was added for suspension, the protoplast concentration was adjusted to 2×10⁵/ml×10⁶/ml by microscopic examination (×100), and placed on the ice for transformation.

### 3. Wheat protoplast transformation

1) 20 µg of plasmid was added to a 2 ml centrifuge tube, 200 µl of the protoplast was added with a spear without a tip and gently mixed uniformly, placed stilly for 3-5 min, and 250 µl of polyethylene glycol (PEG) solution was added, flicked gently and mixed uniformly, transformed in the dark for 30 min.
2) 900 µl of the W5 solution was added and mixed upsidedown uniformly at a room temperature, centrifuged with 80 g for 3 min, and the supernatant was discarded.
3) 1 ml of the W5 solution was added, mixed upsidedown uniformly, and gently transferred to a 6-well plate in which 1 ml of the W5 solution was added per well in advance, and cultured at 23 °C for 24-48 h.

### 4. Detection of amplicon copy number by fluorescent quantitative PCR

After 24-48 h of culture, wheat protoplast DNA was extracted. The residual plasmid DNA was digested with DpnI treatment. PCR system: BIO-RAD iTaq Universal SYBR Green Mix 10 µL, 8.4 µL of diluted DNA template, 0.8 µL of F primer, and 0.8 µL of R primer. qPCR procedure: 95°C 30 s, 95°C 10 s, 60°C 15 s, and 38 cycles. The WDV replicon was amplified with primers WDVLIR-qF/R, and the genomic DNA was amplified with a primer TaPDS-qF/R. The Ct value of qPCR result was converted into the absolute concentration by a standard curve, and then the ratio of the two was calculated to obtain the copy number of a WDV amplicon.

### 5. Cultivation of tobacco plants

A layer of filter paper was placed in a petri dish, and soaked with water, and tobacco seeds were sprinkled on the filter paper, cultured under light at 22°C for about 5 days. Sprouted seedlings were transplanted into a culture bowl, and cultured for 4 weeks under the conditions of 22±2°C, 1000 Lx illuminance and 14~16 h/d of light.

### 6. Agrobacterium-mediated tobacco transient transformation

Agrobacterium tumefaciens with the target plasmid were inoculated into a Luria-Bertani (LB) medium containing kanamycin and rifampicin, shaken and cultured overnight at 28°C. On the next day, 0.3 ml of turbid Agrobacterium bacteria solution was re-inoculated into 6 ml fresh medium, shaken and cultured at 28°C for 4-6 hours. While the bacteria solution was cultured to 0.6-1.0 of optical density (OD), it was centrifuged, bacterial cells were collected, resuspended with tobacco infection solution, OD was adjusted to the target concentration (which should not exceed 1.6), and incubated in the dark at the room temperature for 30 min to 3 h. Flat and healthy tobacco leaves were selected, and the incubated bacteria solution was injected with an injector. Samples were taken for analysis after 48 h-96 h.

### 7. Deep sequencing method

1) The protoplast DNA was extracted, and then the residual plasmid DNA was digested with the DpnI treatment.
2) In Example 1, primers 35Sp-200F and WDV-Rep-150R were used for performing the first round of PCR amplification on DNA. Barcode primers ngs35Sp-300F and ngsWDV-Rep-100R were used for performing the second round of the amplification on a first round PCR product. In Example 2, primers 35Sp-200F and WDV-Rep-100R were used for performing the first round of PCR amplification on DNA. Barcode primers ngs35Sp-250F and ngsWDV-Rep-50R were used for performing the second round of the amplification on a first round PCR product.
3) Second round PCR product was recovered, mixed in an proportion equal for all treatments, sent to the company for creating a library, and the deep sequencing was performed.

Components of each solution used in the above methods are as follows:

### 50 ml of enzymatic hydrolysate

| Reagent | Added amount | Final concentration |
|---|---|---|
| Cellulase R10 | 0.75 g | 1.5% |
| Macerozyme R10 | 0.375 g | 0.75% |
| Mannitol | 5.4651 g | 0.6 M |
| Methyl ethyl sulphate (MES) | 0.1066 g | 10 mM |
| The volume is 50 ml, and the pH value is adjusted to 5.7 by KOH; and incubated in a 55°C water bath for 10 minutes, and added after being naturally cooled at the room temperature. | | |
| CaCl₂ | 0.0735 g | 10 mM |
| Bovine serum albumin (BSA) | 0.05 g | 0.1% |
| Filtered with a 0.45 um filter membrane | | |

### 500 ml of W5

| Reagent | Added amount | Final concentration |
|---|---|---|
| NaCl | 4.5 g | 154 mM |
| CaCl₂ | 9.189 g | 125 mM |
| KCl | 0.1864 g | 5 mM |
| MES | 0.2132 g | 2 mM |
| The volume is 500 ml, and pH is adjusted to 5.7 by NaOH. | | |

### 10 ml of MMG solution

| Reagent | Added amount | Final concentration |
|---|---|---|
| Mannitol (0.8 M) | 5 ml | 0.4 M |
| MgCl₂ (1 M) | 0.15 ml | 15 mM |
| MES (200 mM) | 0.2 ml | 4 mM |
| DDW | To 10 ml | |

### 4 ml of PEG solution

| Reagent | Added amount | Final concentration |
|---|---|---|
| PEG4000 | 1.6 g | 40% |
| Mannitol (0.8 M) | 1 ml | 0.2 M |
| CaCl₂ (1 M) | 0.4 ml | 0.1 M |
| DDW | To 4 ml | |

### 5. Tobacco infection solution

| Reagent | Final concentration |
|---|---|
| MgCl₂ | 10 mM |
| MES | 10 mM |
| Acetosyringone | 150 µM |

### Example 1: Evolution of 20th amino acid of Rep/RepA achieved by using plant directed evolution system based on geminivirus

The 20th amino acid of wild-type WDV Rep/RepA is a tyrosine, and this amino acid is highly conservative in this genus. Previous research show that the mutant Rep/RepA Y20C of Rep does not have the replication initiation activity. In order to verify whether the directed evolution system of the present invention can work, the inventors firstly attempted to evolve the 20th amino acid of Rep/RepA.

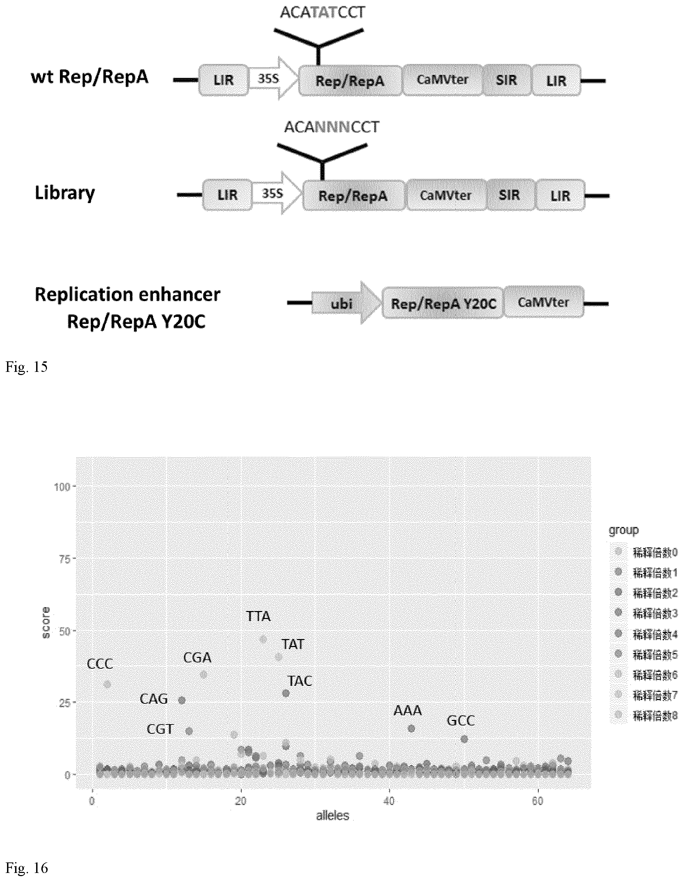

Firstly, a codon of the 20th amino acid of Rep/RepA was converted from TAT to NNN by a PCR method, and then a library with diversity of 64 (43) was obtained by cloning a PCR fragment onto the geminivirus vector (as shown in Fig. 15). Then, the library was transformed into wheat protoplasts at different concentration gradients (10 µg-0.1 ng, divided into 10 concentration gradients). After 48 h, protoplast DNA was extracted, and deeply sequenced for the site. Sequencing results of each concentration were compared with the results of the initial library.

It was found from the results that 12 of 64 alleles contained in the library were enriched, encoding proline, glutamine, arginine, leucine, tyrosine, lysine and alanine respectively (as shown in Fig. 16). 35% of the encoded amino acids generated positive screening, which did not meet the initial expectations. It was speculated that the reason is that the expression quantity of Rep/RepA is positively correlated with the copy number of the rolling-circle replication, and while the used amount of the library in protoplast transformation is gradually reduced, the proportion of cells transformed with only one molecule is increased, but then the expression quantity of Rep/RepA expressed by the single molecule is relatively low, it is not enough to drive the replicon to generate the high-efficient rolling-circle replication. However, if the used amount of the library is relatively high, the probability of a functional allele and a non-functional allele co-transformed into the same cell is increased, and it may cause the amplification of the non-functional allele. This may results in a very high background noise.

Based on this result, the inventor hoped to find a replication enhancer. On the one hand, this replication enhancer may not independently initiate the rolling-circle replication, and on the other hand, while the Rep/RepA expression quantity is relatively low, the existence of this replication enhancer may greatly increase the copy number of the replicon. The previous research show that Rep/RepA of the geminivirus is a multifunctional protein, and in addition to initiating the rolling-circle replication, it is also an inhibitor of post transcriptional gene silencing (PTGS), and a transcription activator of a viral coding gene, and may interact with an endogenous protein, allowing a mature cell has the ability of high level DNA replication again, and the like. Based on this assumption, the inventor attempted to find a mutant of Rep/RepA. On the one hand, it may no longer initiate the rolling-circle replication, and on the other hand, it retains other functions except for initiating the rolling-circle replication, so that it can be used as the replication enhancer.

Firstly, the inventor constructed several Rep/RepA mutants, including Y106H, K229E, Y20C, H59R, E198A and H91R, and then the replication initiation activity of these mutants were detected by the fluorescent quantitative PCR. Results show that except H91R, all other mutants have no replication initiation activity at all. Then, in order to screen the suitable replication enhancer, the added amount of the Rep/RepA plasmid was reduced from original 10 µg to 100 ng, and at this concentration, the copy number of the replicon may only reach a lower level. In this case, the addition of the replication enhancer should be able to greatly increase the level of the copy number. After being detected, it was found that RepA, Rep/RepA K229E and Y20C may be used as the replication enhancer. In subsequent experiments, Rep/RepA Y20C is used as the replication enhancer.

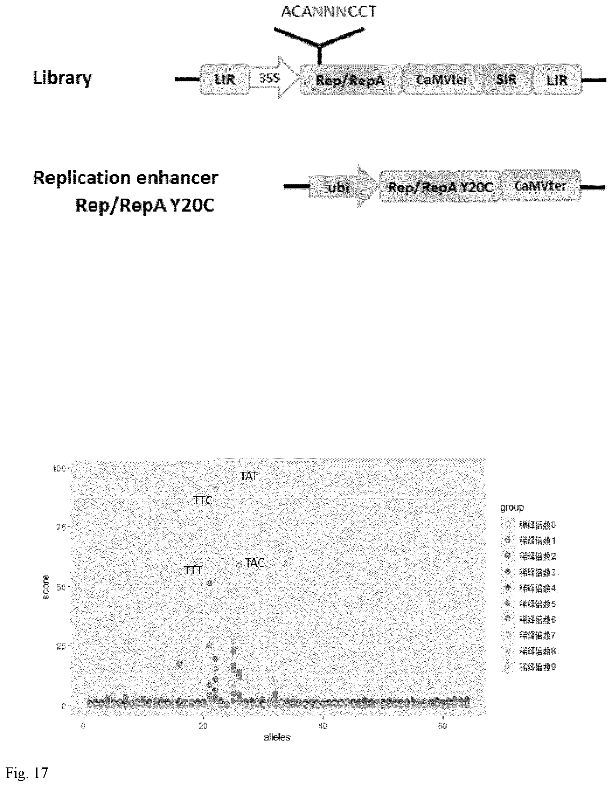

The evolution experiment of the 20th codon of Rep/RepA was performed again. It was found that after the replication enhancer was added, 4 alleles were enriched, encoding phenylalanine and tyrosine respectively (as shown in Fig. 17). In addition to the expected tyrosine, the phenylalanine was also enriched. It is specifically speculated that the reason is that a motif of Rep/RepA Y20 needs to interact with DNA, and the phenylalanine, like the tyrosine, has an aromatic residue which may generate π - π stacking with a DNA base. This work shows that the system of the present invention may work.

In this experiment, it was found that the amount of the library added is also a key factor affecting the evolution system. In a series of the concentration gradients, it was found that while the dilution ratio reaches 5-10, namely the added amount is about 1×10⁻¹³ mol to 1×10⁻¹⁵ mol, which is 10³ to 10⁵ times of the protoplast amount, the screening effect is the best.

### Example 2: Evolution of CaMV 35S promoter TATA-box achieved by using plant directed evolution system based on geminivirus

CaMV 35S promoter is a constitutive promoter commonly used in plants. There is a TATA-box motif at about 30 bp upstream of its transcription initiation site, and the sequence of which is 5'-ctatataag-3'. Most eukaryotic pol type-II promoters have a TATA-box element, and it is critical to the transcriptional activity of the promoter. In this Example, it was intended to evolve the CaMV 35S promoter TATA-box, and simultaneously study the effect of the element sequence on the activity of the CaMV 35S promoter.

Firstly, the activity of the CaMV 35S promoter was coupled with the expression of Rep/RepA. The CaMV 35S promoter was used to drive expression of WDV Rep/RepA, and the two were placed in the replicon together.

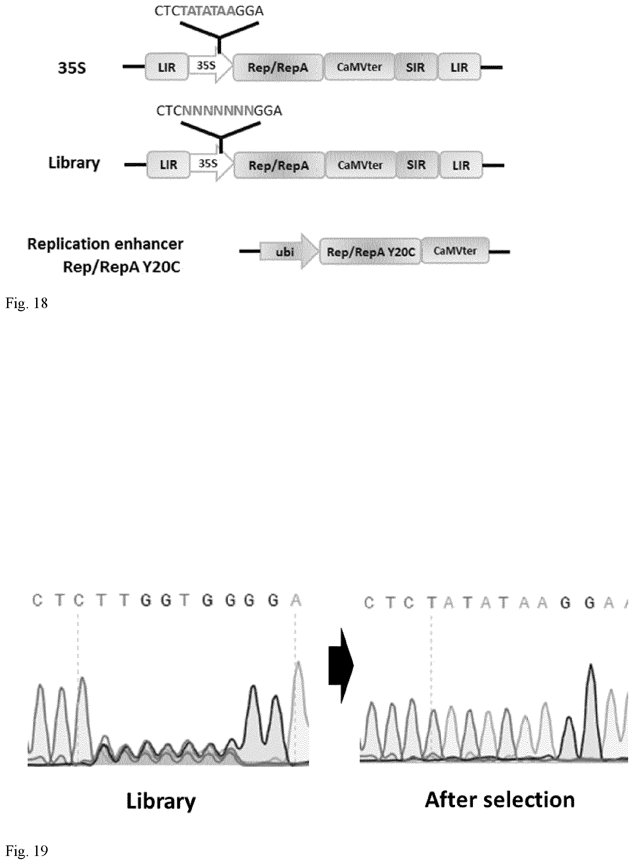

The second step was to construct a screening library. TATA-box of the CaMV 35S promoter was converted from CTATATAAG to CNNNNNNNG by PCR method, and then the PCR fragments were cloned onto the geminivirus vector to obtain a library with theoretical diversity of 16384 (4⁷) (as shown in Fig. 18). Then, the library was transformed into wheat protoplasts at the different concentrations. After 48h, protoplast DNA was extracted, and the site was sequenced. Sequencing results of each concentration were compared with the results of the initial library.

It was found from the results by Sanger sequencing and amplicon sequencing that, in the library of which the sequence is CNNNNNNNG, after screening, a sequence corresponding to the original TATA-box was enriched, so that almost all the sequences become CTATATAAG (as shown in Fig. 19). It was consistent with the expected results.

### Example 3: Rapid screening of pegRNA by using directed evolution system based on primary replicon

Prime editing system is a gene editing system that may perform any base replacement and small fragment deletion or insertion, and it includes two portions, namely a nCas9 (H840A) protein fused with an M-MLV reverse transcriptase and a prime editing guide RNA (pegRNA). The prime editing system may work more efficiently in yeast and animal cells, but it is very inefficient in the plants and has very strong site specificity. pegRNA includes 4 portions, a spacer portion responsible for recognizing a target site, a scaffold part responsible for linking with nCas9, a primer binding site (PBS) as a primer responsible for complementing with 5' end sequence of a nCas nick, and a reverse transcription template (RT) portion, as a reverse transcription template, responsible for repairing the 3' end of the nick into an given sequence. For one target site, Spacer and Scaffold regions are fixed, but PBS and RT regions are highly variable, and the length and sequence of the two regions have a great impact on the efficiency of prime editing. Therefore, it is hoped to establish a high-throughput plant system capable of screening pegRNAs.

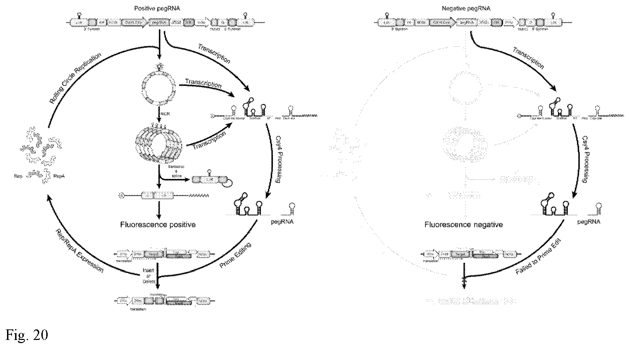

Firstly, the function of pegRNA needs to be coupled with the expression of Rep/RepA. The target site is inserted at N end of Rep/RepA, resulting in frame-shifting of the open reading frame of Rep/RepA. While the pegRNA is absent or ineffective, Rep/RepA cannot be expressed. However, if the pegRNA is active, it may introduce short insertion or deletion at the target site, so that Rep/RepA may be correctly expressed. Based on this principle, an expression cassette of pegRNA is inserted into the geminivirus replicon, and a fluorescence reporter system is inserted at its two ends (as shown in Fig. 20). If the virus replicon generates the rolling-circle replication under the action of pegRNA, a fluorescence signal is reported; and if the virus replicon does not generate the rolling-circle replication, there is no fluorescence signal.

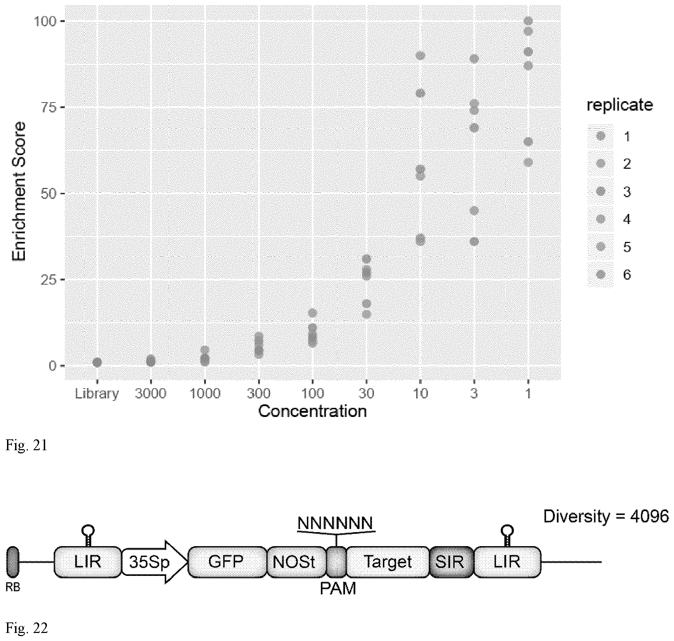

In order to verify whether the system works, two vectors were constructed, wherein one vector contains known pegRNA with activity, and the other contains a mutation in PBS of pegRNA which results in loss of activity. The two vectors were mixed in an equal proportion to form a library and transformed into tobacco leaves at different concentrations. After 6 days, DNA was extracted and detected, and it was found by the inventors while the tobacco leaves were transformed with a high concentration of library, pegRNA with activity was not enriched; and while the tobacco leaves were transformed with a low concentration of library, pegRNA with activity was significantly enriched (as shown in Fig. 21). This meets the expectations and proves that the system may work.

### Example 4: Rapid identification of PAM of Cas protein by using directed evolution system based on secondary replicon

In the field of genome editing, researchers already found many Cas proteins with nuclease activity (including Cas9, Cas12a, Cas12b and the like). One of the characteristics of these Cas proteins is that there needs to be a specific sequence at upstream or downstream of the cutting target site, called protospacer adjacent motif (PAM). Different Cas proteins have different PAM sequences.

In previous researches, a Cas12a protein, called FbCas12a, was found in Flavobacterium branchiophellum by using a method of bioinformatics analysis. In order to apply it in biotechnologies, it is necessary to determine its PAM sequence firstly. For this reason, a library containing 4096 PAMs was constructed (as shown in Fig. 22).

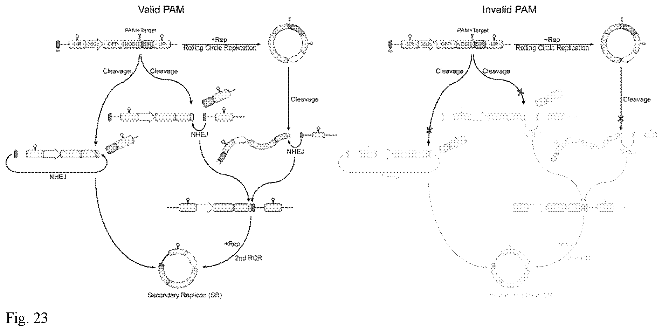

If a certain PAM can be recognized by FbCas12a, the latter may generate DSB in the target site region and then form a secondary replicon, and PAM information may remain in the secondary replicon; and if a certain PAM cannot be recognized by FbCas12a, DSB cannot be generated and PAM cannot be retained in the secondary replicon. Therefore, as long as the secondary replicon is specifically detected, it can be known which PAM can be recognized by FbCas12a (as shown in Fig. 23).

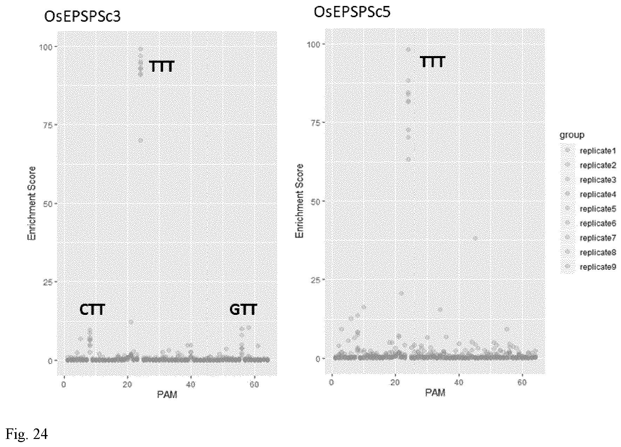

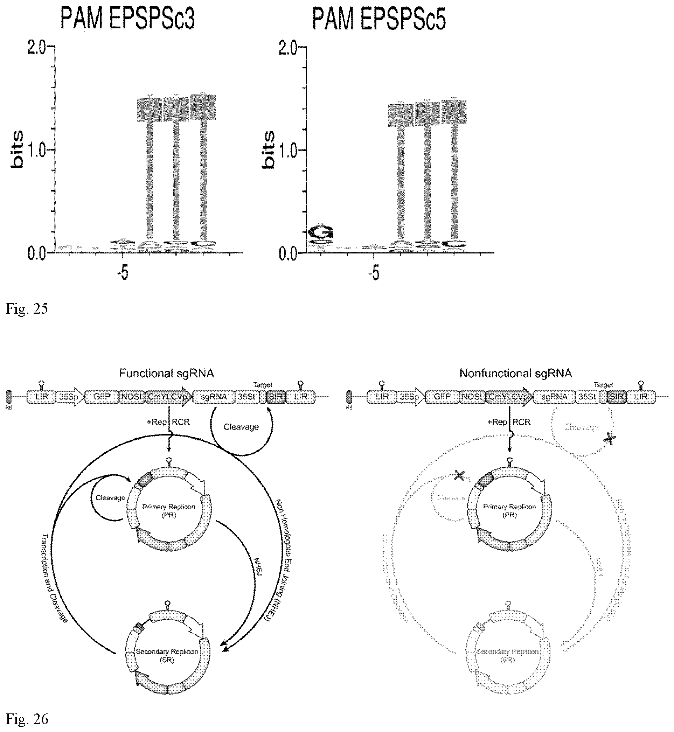

Two target sites, OsEPSPSC3 and c5, were selected for testing. After testing, it was apparent that PAM that can be recognized by FbCas12a is 'TTT' (as shown in Fig. 24). At other positions, no apparent base preference was found (as shown in Fig. 25).

### Examole 5: Rapid screening of sgRNA by using directed evolution system based on secondary replicon

For a Cas protein, it needs a RNA to guide its nucleic acid cutting, called sgRNA (Cas9) or crRNA (Cas 12a, Cas 12b). Its sequence and structure greatly affect the activity of the Cas protein. Therefore, it is hoped to establish a system that can screen sgRNA quickly in high throughput.

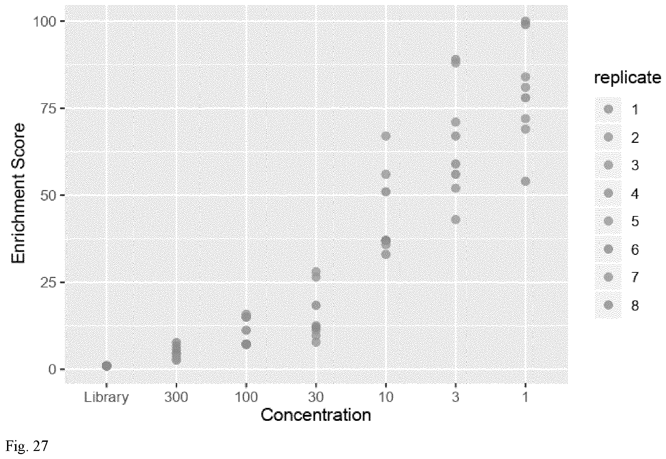

Similar to Example 4, vectors shown in the figure are designed (as shown in Fig. 26). If a certain sgRNA is active, it can guide Cas9 to cut the target site located at its downstream, thereby a secondary replicon is formed; and if a certain sgRNA is inactive, it cannot generate DSB, and the secondary replicon cannot be generated. Based on this principle, two vectors were constructed, wherein one contains a sgRNA with activity, and the other containing a sgRNA without the activity. After 5 days of screening, it was found that sgRNA with activity in the secondary replicon was significantly enriched, and enrichment was related to the library concentration (as shown in Fig. 27). This proves that the system works.

### Sequence listing

>SEQ ID NO 1 WDV-LIR 
GGTAGTGAACAGAAGTCCGGCAGGTCCTTAGCGAAAAAACGGGGTGTGCCAGAAAAC
TCTATCCTCTACCCTGCGTGGAGGTGTGAATTCTGCACACTGCAAATGCAATGTGTCCA
ATGCTTTATATAGGGCAGGTTTTGGCGGGAGAACAGGGCCCTAGTGTTCCCACGGTAGC
GTAGCGAATCGTGTGGGCCCTGTTCGGTGTGCGGTCGGGGGGCCTCCACGCGGGTTAT
AATATTACCCCGCGTGGTGGCCCCCGACGCGCACTCGGCTTTTCGTGAGTGCGCGGAG
GCTTTTGGACCACATCTTTTCTGATCACTTTCGTGGAAGATGTTGATTTATCACACTTTT
GACGGGGAAATCTGTGCCATGCCTTAGCTTATAAGGAAGTGCGTGGTAGCCCATCTCG
>SEQ ID NO 2 WDV-SIR 
TAAAATAATATTTTATTTATCTCATGTCATTCGATTACAGAGGCTCGGCTACGAGCAAAG
ACAAACCAAATATAACAAACAACAACCCTTACACAATGACATCGGAAAACGAAATACA
ACACCCTGAGATATTACATTTATAGAAACTGTACGCCGTCCGCGCTAGGACAG
>SEQ ID NO 3 WDV-Rep 
MASSSAPRFRVYSKYLFLTYPQCTLEPQYALDSLRTLLNKYEPLYIAAVRELHEDGSPHLH
VLVQNKLRASITNPNALNLRMDTSPFSIFHPNIQAAKDCNQVRDYITKEVDSDVNTAEWG
TFVAVSTPGRKDRDADMKQIIESSSSREEFLSMVCNRFPFEWSIRLKDFEYTARHLFPDPVA
TYTPEFPTESLICHETIESWKNEHLYSESPGRHKSIYICGPTRTGKTSWARSLGTHNYYNSL
VDFTTYDVNAKYNIIDDIPFKFTPNWKCFVGAQRDFTVNPKYGKRKVIRGGIPCIILVNPDE
DWLKDMTPEQSDYMYSNTVVHYMYEGETFINYSFASGEDVTASQ*
> SEQ ID NO 4 WDV-Rep Y20C 
MASSSAPRFRVYSKYLFLTCPQCTLEPQYALDSLRTLLNKYEPLYIAAVRELHEDGSPHLH
VLVQNKLRASITNPNALNLRMDTSPFSIFHPNIQAAKDCNQVRDYITKEVDSDVNTAEWG
TFVAVSTPGRKDRDADMKQIIESSSSREEFLSMVCNRFPFEWSIRLKDFEYTARHLFPDPVA
TYTPEFPTESLICHETIESWKNEHLYSESPGRHKSIYICGPTRTGKTSWARSLGTHNYYNSL
VDFTTYDVNAKYNIIDDIPFKFTPNWKCFVGAQRDFTVNPKYGKRKVIRGGIPCIILVNPDE
DWLKDMTPEQSDYMYSNTVVHYMYEGETFINYSFASGEDVTASQ*
> SEQ ID NO 5 WDV-RepA 
MASSSAPRFRVYSKYLFLTYPQCTLEPQYALDSLRTLLNKYEPLYIAAVRELHEDGSPHLH
VLVQNKLRASITNPNALNLRMDTSPFSIFHPNIQAAKDCNQVRDYITKEVDSDVNTAEWG
TFVAVSTPGRKDRDADMKQIIESSSSREEFLSMVCNRFPFEWSIRLKDFEYTARHLFPDPVA
TYTPEFPTESLICHETIESWKNEHLYSVSLESYILCTSTPADQAQSDLEWMDDYSRSHRGGI
SPSTSAGQPEQERLPGQGL*
> SEQ ID NO 6 WDV-RepA Y20C

MASSSAPRFRVYSKYLFLTCPQCTLEPQYALDSLRTLLNKYEPLYIAAVRELHEDGSPHLH
VLVQNKLRASITNPNALNLRMDTSPFSIFHPNIQAAKDCNQVRDYITKEVDSDVNTAEWG
TFVAVSTPGRKDRDADMKQIIESSSSREEFLSMVCNRFPFEWSIRLKDFEYTARHLFPDPVA
TYTPEFPTESLICHETIESWKNEHLYSVSLESYILCTSTPADQAQSDLEWMDDYSRSHRGGI
SPSTSAGQPEQERLPGQGL*

## Claims

1. A method for directed evolution of a genetic element to obtain a mutant of the genetic element with a desired function, wherein the method comprises:
i) providing a library of the mutants of the genetic element, wherein it contains a plurality of the mutants of the genetic element respectively inserted into a vector containing a geminivirus replicon, and wherein the mutant is inserted into the geminivirus replicon so that while the geminivirus replicon is replicated, the mutant is amplified,
ii) transforming a population of plant cells with the library, and
iii) culturing the population of plant cells, detecting and selecting the genetic element mutant enriched in the population of plant cells,
wherein the replication level of the geminivirus replicon in the plant cells is configured to be associated with the desired function of the genetic element mutant.

2. The method according to claim 1, wherein the genetic element is selected from a protein coding sequence; a functional RNA coding sequence, such as tRNA and siRNA coding sequences; and an expression regulatory sequence such as a promoter sequence, an enhancer sequence, and a terminator sequence.

3. The method according to claim 1 or 2, wherein the genetic element is derived from a plant, or is expected to be applied in a plant.

4. The method according to any one of claims 1-3, wherein the library of the mutants of the genetic element is obtained by respectively inserting the plurality of the mutants of the genetic element into a vector containing the geminivirus replicon.

5. The method according to claim 4, wherein the plurality of the mutants of the genetic element is generated by random mutagenesis of the genetic element.

6. The method according to any one of claims 1-3, wherein the library is generated by performing random mutagenesis on the genetic element that has been inserted into the vector containing the geminivirus replicon.

7. The method according to any one of claims 1-6, wherein the geminivirus is a wheat dwarf virus (WDV).

8. The method according to any one of claims 1-6, wherein the geminivirus is a bean yellow dwarf virus (BeYDV).

9. The method according to any one of claims 1-8, wherein the vector containing the geminivirus replicon is a circular DNA, such as a plasmid or a minicircle DNA.

10. The method according to any one of claims 1-9, wherein the vector containing the geminivirus replicon contains at least one large intergenic region (LIR), for example, the LIR comprises the nucleotide sequence shown in SEQ ID NO: 1.

11. The method according to any one of claims 1-10, wherein the vector containing the geminivirus replicon contains at least one small intergenic region (SIR), for example, the SIR comprises the nucleotide sequence shown in SEQ ID NO: 2.

12. The method according to any one of claims 1-11, wherein the vector containing the geminivirus replicon contains one LIR.

13. The method according to any one of claims 1-11, wherein the vector containing the geminivirus replicon contains two LIRs.

14. The method according to any one of claims 1-13, wherein in the vector containing the geminivirus replicon, the inserted mutant of the genetic element is operably linked with an expression regulatory sequence.

15. The method according to any one of claims 1-14, wherein the vector containing the geminivirus replicon further contains an expression cassette of a geminivirus Rep and/or RepA protein.

16. The method according to any one of claims 1-14, wherein the vector containing the geminivirus replicon does not contain an expression cassette of a geminivirus Rep and/or RepA protein.

17. The method according to claim 16, wherein the method further comprises introducing another vector for expressing the geminivirus Rep and/or RepA protein into the plant cells, for example, the population of plant cells are co-transformed with the another vector for expressing a geminivirus Rep and/or RepA protein together with the library.

18. The method according to claim 16, wherein the plant cell already contains a vector for expressing a geminivirus Rep and/or RepA protein, and/or the genome of the plant cell is already integrated with an expression cassette of a geminivirus Rep and/or RepA protein.

19. The method according to any one of claims 1-18, wherein the geminivirus Rep protein comprises the amino acid sequence shown in SEQ ID NO: 3, or comprises an amino acid sequence with amino acid substitution K229E or Y20C relative to SEQ ID NO: 3, preferably comprises the amino acid sequence shown in SEQ ID NO: 4.

20. The method according to any one of claims 1-18, wherein the geminivirus RepA protein comprises the amino acid sequence shown in SEQ ID NO: 5, or comprises an amino acid sequence with amino acid substitution K229E or Y20C relative to SEQ ID NO: 5, preferably comprises the an amino acid sequence shown in SEQ ID NO: 6.

21. The method according to any one of claims 1-20, wherein the activity or expression level of the geminivirus Rep and/or RepA protein in the plant cell is configured to be associated with the desired function of the genetic element mutant.

22. The method according to any one of claims 1-21, wherein in the transformation of the step ii), the number of vector molecules containing the mutants in the library is 10³ to 10⁵ times of the number of the cells in the population of plant cells.

23. The method according to any one of claims 1-22, wherein the expression or activity of the geminivirus Rep and/or RepA protein in the plant cell is coupled with the desired function of the genetic element mutant, thereby the directed evolution of the genetic element is achieved.

24. The method according to any one of claims 1-23, wherein the genetic element mutant with the desired function activates Rep/RepA expression, drives the rolling-circle replication and thus achieves self-enrichment; while the genetic element mutant without the desired function cannot activate the Rep/RepA expression such that enrichment cannot be achieved.

25. The method according to claim 1, wherein the genetic element is a promoter.

26. The method according to claim 25, wherein the method further comprises placing a promoter library to be evolved to upstream of Rep/RepA in the replicon.

27. The method according to claim 25 or 26, wherein the genetic element is a cauliflower mosaic virus (CaMV) 35S promoter TATA-box.

28. The method according to claim 1, wherein the genetic element is a sequence encoding a transcription activator.

29. The method according to claim 28, wherein the method further comprises inserting a recognition sequence of the transcription activator to upstream of Rep/RepA, and inserting a minimal promoter between the recognition sequence and the Rep/RepA; and placing a library of the transcription activator to be evolved in the replicon.

30. The method according to claim 1, wherein the genetic element is a DNA binding domain.

31. The method according to claim 30, wherein the method further comprises inserting a target binding sequence of the DNA binding domain to upstream of Rep/RepA, and inserting the minimal transcription initiation element between the target binding sequence and Rep/RepA; and placing a fusion protein formed by the DNA binding domain to be evolved and a transcription activator without sequence specificity in the replicon.

32. The method according to claim 1, wherein the genetic element is a sequence encoding a recombinase.

33. The method according to claim 32, wherein the method further comprises dividing Rep/RepA into two portions which are placed at two ends of a recombinase recognition sequence; and placing the sequence encoding the recombinase to be evolved in the replicon.

34. The method according to claim 32 or 33, wherein the method further comprises adding a 5' intron and a 3' intron between Rep/RepA and the recombinase recognition sequences.

35. The method according to claim 1, wherein the genetic element is a prime editing guide RNA (pegRNA).

36. The method according to claim 35, wherein the method further comprises inserting a target site at N terminal of Rep/RepA which results in frame-shifting of the open reading frame of Rep/RepA; and inserting an expression cassette of pegRNA into the geminivirus replicon, and inserting a fluorescence reporter system to two ends thereof.

37. The method according to claim 1, wherein the desired function of the genetic element is coupled with expression of a nuclease.

38. The method according to claim 37, wherein the nuclease is a specific nuclease.

39. The method according to claim 37 or 38, wherein the genetic element with the desired function activates the expression of the nuclease or guides the nuclease to cut its recognition site to allow the rolling-circle replication to achieve the self-enrichment; and the genetic element without the desired function cannot allow the nuclease to cut its recognition site to achieve the enrichment, thereby the directed evolution of the genetic element is achieved.

40. The method according to any one of claims 37-39, wherein the genetic element is a DNA binding domain.

41. The method according to claim 40, wherein the method further comprises fusing a the DNA binding domain to be evolved to a non-sequence specific nuclease, and placing it in the replicon together with its recognition sequence.

42. The method according to any one of claims 37-39, wherein the genetic element is a sequence encoding a non-sequence specific nuclease.

43. The method according to any one of claims 37-39, wherein the genetic element is a sequence encoding a transcription activator.

44. The method according to claim 43, wherein the method further comprises inserting a recognition sequence of the transcription activator to upstream of the nuclease, and inserting a minimal promoter between the recognition sequence and the nuclease; and placing a library of the transcription activator to be evolved in the replicon together with the recognition sequence of the nuclease.

45. The method according to any one of claims 37-39, wherein the genetic element is a sequence encoding a recombinase.

46. The method according to claim 45, wherein the method further comprises placing a library of the recombinase to be evolved and the recognition sequence of a nuclease in the replicon; and dividing the nuclease into two portions which are placed at two ends of the recombinase recognition sequence.

47. The method according to claim 45 or 46, wherein the method further comprises adding a 5' intron and a 3' intron between the nuclease and the recombinase recognition sequence.

48. The method according to any one of claims 37-39, wherein the genetic element is a protospacer adjacent motif (PAM) of a Cas protein.

49. The method according to claim 48, wherein the method further comprises placing sgRNA to be evolved and the target sequence of the Cas protein in the replicon together.

50. The method according to any one of claims 37-39, wherein the genetic element is sgRNA.

51. The method according to claim 50, wherein the method further comprises placing sgRNA to be evolved and the target sequence of the Cas protein in the replicon together.

52. The method according to any one of claims 1-51, wherein in step iii), the detecting and selecting of the genetic element mutant enriched in the population of plant cells is performed by high-throughput sequencing.

53. The method according to any one of claims 1-52, wherein it further comprises a step iv) of identifying the function of the enriched genetic element mutant.

54. The method according to any one of claims 1-53, wherein the plant is a monocotyledon or a dicotyledon, for example, it is selected from corn, wheat, rice, barley, sorghum, kidney bean, beet, tomato, cassava, cucumber, arabidopsis and tobacco.
